# EUROPEAN PATENT SPECIFICATION

(11) **EP 0 880 970 B1**
(45) Date of publication and mention of the grant of the patent: **02.08.2006**
(21) Application number: 98104718.6
(22) Date of filing: 07.05.1992
(51) Int. Cl.: A61K 38/17, A61P 37/00

(54) **Use of pharmaceutical compositions comprising an anticytokine for the therapy of multiple sclerosis**
Verwendung von Antizytokin-enthaltenden pharmazeutische Zusammensetzungen zur Therapie von multipler Sklerose
Utilisation de compositions pharmaceutiques comprenant un anticytokine pour la thérapie de la sclérose multiple

(30) Priority: 07.05.1991 IL 9807891
(43) Date of publication of application: 02.12.1998
(62) Divisional of application: 92107685.7
(73) Proprietor: YEDA RESEARCH AND DEVELOPMENT COMPANY LIMITED, Rehovot 76100 (IL)
(72) Inventor: Wallach, David, Rehovot (IL); Aderka, Dan, Holon (IL); Engelmann, Hartmut, D-81371 München (DE)
(74) Representative: Vossius & Partner

(56) References cited:
- EP-A- 0 308 378
- EP-A- 0 393 438
- EP-A- 0 398 327
- EP-A- 0 412 486
- EP-A- 0 433 900
- WO-A-92/07076
- GB-A- 2 246 569
- ADERKA ET AL: "THE POSSIBLE ROLE OF TUMOR NECROSIS FACTOR (TNF) AND ITS NATURAL INHIBITORS, THE SOLUBLE-TNF RECEPTORS, IN AUTOIMMUNE DISEASES" ISRAEL JOURNAL OF MEDICAL SCIENCES / FIFTH AUTOIMMUNITY MEETING OF THE ISRAEL IMMUNOLOGICAL SOCIETY. TEL-HASHOMER. ISRAEL. MAY 7. 1991, vol. 28, no. 2, February 1992, pages 126-130, XP002079556 JERUSALEM, ISRAEL

## Description

The present invention relates to the use of a Tumor Necrosis Factor (TNF) Binding Protein, herein designated TBP, for the manufacture of pharmaceutical compositions for the treatment of multiple sclerosis.

Tumor Necrosis Factor (TNF) is a multifunctional cytokine involved in the protection of the organism, but when overproduced it can play a major pathogenic role in several diseases. TNF is known to be involved in inflammatory processes and to be a mediator of the damage to tissues in rheumatic diseases (Beutler, B. and Cerami, C. (1987) NEJM 316:379-385) and of the damage observed in graft-versus-host reactions (Piguet, P.F. et al. (1987) J. Exp. Med. 166:1280-89).

Two TNF Binding Proteins, designated TBP-I and TBP-II were first described in Israel Patent applications No. 83878 and 90339, or the corresponding European Patent Applications EP-A-308 378 and EP-A-398 327, respectively, of the same applicant, and shown to protect cells from TNF toxicity and to interfere with the binding of TNF to cells. Later studies have shown that these two proteins are structurally related to two molecular species of the cell surface TNF receptors (TNF-R) and that, indeed, TBP-I is related to a soluble form of the TNF type I receptor, while TBP-II is related to a soluble form of the TNF type II receptor (Engelmann, H. et al. (1989) J. Biol. Chem. 264:11974-11980; Engelmann, H. et al. (1990) J. Biol. Chem. 265:1531-1536). Like the cell surface receptors for TNF, the soluble forms of these receptors specifically bind TNF and can thus interfere with its binding to cells, functioning as physiological inhibitors of TNF activity.

Although the primary function of the immune system is to protect an individual against infection by foreign invaders such as microorganisms, it may happen that the immune system attacks the individual's own tissues, leading to pathologic states known as autoimmune diseases, which are frequently associated with inflammatory processes. Examples of autoimmune diseases are rheumatoid arthritis, juvenile onset type I diabetes mellitus, systemic lupus erythematosus, thyroiditis and multiple sclerosis. Rheumatoid arthritis is a disease marked by signs and symptoms of inflammation of the joints. Systemic lupus erythematosus (SLE) is characterized by red, scaley patches on the skin and by malfunction of the kidneys at the advanced stage of the disease, and is associated with inflammatory reactions triggered by deposition of immune complexes in blood vessels, particularly in the kidneys. Multiple sclerosis is a human illness characterized by relapsing, inflammatory conditions that can cause weakness, body tremors and, in extreme cases, paralysis, and is associated with immune system attack of the protective myelin sheath surrounding peripheral nerve cells. TNF has been associated with inflammatory processes in systemic lupus erythematosus, rheumatoid arthritis and multiple sclerosis.

In published European patent applications of the same applicant No. 398327 and 412486, it is disclosed that in SLE patients the serum levels of both TBP-I and TBP-II are significantly elevated and in correlation with the disease activity, indicating that TBP-I and TBP-II may be used as sensitive markers of the disease activity and may be useful in monitoring immune activation related to disease activity in SLE patients as well as in patients with other autoimmune diseases.

It was now found, according to the present invention, that Tumor Necrosis Factor Binding Proteins are useful in the treatment of multiple sclerosis. It is believed that the TBPs complement the physiological activity of the endogenous soluble TNF receptors, types I and II, whose formation in autoimmune diseases like multiple sclerosis is suggested to constitute a safeguard mechanism against over-response to the damaging effects of TNF.

Accordingly, the present invention provides the use of a Tumor Necrosis Factor Binding Protein, herein designated TBP, a salt, a functional derivative, a precursor or an active fraction thereof, or combinations of the foregoing, for the manufacture of pharmaceutical compositions, for the treatment of multiple sclerosis.

The TBPs for use in the compositions of the present invention may be obtained from natural sources, such as human urine (Engelmann, H. et al. (1989) J. Biol. Chem. 264:11974-11980; Engelmann, H. et al. (1990) J. Biol. Chem. 265:1531-1536; Olson, I. et al., (1989) Eur. J. Haematol. 42:270-275; Seckinger, P. et al., (1989) J. Biol. Chem. 264: 11966-11973) or by recombinant techniques (Nophar, Y. et al., (1990) EMBO J. 9:3269-3278; Schall, T.J. et al., (1990) Cell. 61:361-370; Loetscher, H. et al., (1990) Cell 61:351-359) and then further purified as described in the above-mentioned Israel Patent Applications No. 83878 and 90339.

As used herein, the terms "TBPs", "TBP-I" and "TBP-II" refer to all TNF Binding Proteins from natural sources or obtained by recombinant DNA techniques, including but not limited to the TNF Binding Proteins I and II described in IsraelPatent applications 83878 and 90339, as well as to the soluble forms of the cell surface TNF receptors types I and II, and salts, functional derivatives, precursors and active fractions of the foregoing, these last definitions being as defined in Israel Patent applications 83878 and 90339.

The term "pharmaceutically acceptable" is meant to encompass any carrier that does not interfere with the effectiveness of the biological activity of the active ingredient and that is not toxic to the host to which it is administered. For example, for parenteral administration, the TBP may be formulated in a unit dosage form for injection in vehicles such as saline, dextrose solution, normal serum albumin and Ringer's solution. Any mode of parenteral administration may be suitable, including intravenous, intramuscular and subcutaneous administration. Local administration may be preferred, however, if local inflammation is to be treated, e.g. local injection to treat joint inflammation in rheumatoid arthritis, or injection into the cerebrospinal fluid in multiple sclerosis. Besides the pharmaceutically acceptable carrier, the compositions of the invention will also comprise minor amounts of additives, such as stabilizers, excipients, buffers and preservatives.

The term "effective amount" refers to an amount of TBP that is sufficient to affect the course and severity of the autoimmune disease and to improve the patient's condition, leading to reduction or remission of the disease. The effective amount will depend on the route of administration, the disease to be treated and the condition of the patient. Determination of the level of TBP-I and TBP-II in the serum or other suitable body fluid of the patient, may help to establish a suitable dose for said patient, considering that the exogenously administered TBP may complement the endogenously formed TBP in neutralizing the TNF deleterious activity.

The invention will be illustrated by the following examples. In some of the examples, animal models of experimental autoimmune diseases are employed (Cohen, I.R. (1985) J.. Invest. Dermatol. 85:34s-38s)

### Reference example 1: Treatment of adjuvant arthritis in rats

Adjuvant arthritis is an experimental disease characterized by chronic inflammation of the joints, inducible in certain strains of rats by immunization with complete Freund's adjuvant or with fractions of Mycobacterium tuberculosis, and is considered to be a model of human rheumatoid arthritis (Pearson, C.M. (1964) Arthritis Rheum. 7:80-86). The disease appears about 11-12 days after immunization, and is characterized by mononuclear cell infiltration of the synovia, most prominent in the small joints of the extremities, with panus formation, a process that may progress for months resulting in destruction of bones and ankylosis of joints. Lewis rats are immunized with M. tuberculosis (1mg) in oil to induce adjuvant arthritis (Pearson, C.M. (1956) Proc. Soc. Exp. Biol. Med. 91:95-101). Some days later, before or after the onset of overt clinical arthritis, the rats are innoculated subcutaneously with different doses of TBP-I or TBP-II once or daily for several days, and then scored for the development of arthritis on a scale of 0-16 as described (Holoshitz, Y. et al., (1983) Science, 219:56-58). Doses that inhibit the appearance or produce a partial inhibition of disease are effective doses. Optimal doses are those administered after onset of the disease that suppress the course and cause a permanent remission of the disease. Suitable doses for human patients can be calculated from these doses.

### Example 2: Treatment of experimental autoimmune encephalomyelitis (EAE) in rats

Experimental autoimmune encephalomyelitis (EAE) is an experimental disease inducible in a variety of species: rats, guinea pigs, mice, rabbits, etc. by immunization with white matter of the central nervous system or with the basic protein of myelin or a fragment thereof. It is considered to be a model of multiple sclerosis and, similar to this neurological human disorder, EAE is an autoimmune disorder in which the immune system attacks the protective myelin sheath surrounding peripheral nerve cells. The disease is characterized clinically by acute paralysis and histologically by mononuclear cell infiltrates around blood vessels in the white matter of the central nervous system (Cohen, I.R., see above). Rats are injected with guinea-pig BP or the major encephalitogenic fragment of BP (amino acids 68-88) in a suitable adjuvant such as complete Freund's adjuvant to induce EAE. One day before innoculation and daily for ten days, the rats receive either saline (control) or different doses of TBP-I or TBP-II. The rats are observed for development of paralysis. Doses inhibiting the severity of disease are to be considered effective doses.

### Reference example 3: Correlation between serum levels of TBP-I and TBP-II and anti-dsDNA antibodies in SLE patients

The levels of TBP-I and TBP-II were determined in the sera of 38 SLE patients and 140 healthy controls by the ELISA method described in published European patent Applications No. 398327 and 412486. The serum concentrations (mean±SD) of TBP-I and TBP-II in the control group were 0.77±0.19 ng/ml and 3.02±0.57 ng/ml, respectively. These values were independent of age and sex. In the SLE patients, significantly higher concentrations of TBP-I and TSP-II were observed. The mean ±SD concentrations were, for TBP-I 1.89±0.89 ng/ml and for TBP-II 7.25±3.89 ng/ml.

The results were compared to the levels of anti-dsDNA antibodies, a parameter considered as a reliable and sensitive indicator of the SLE disease activity. Close examination of the extent of the correlation of the TBPs with the anti-dsDNA antibodies in individual patients revealed 3 distinctive subgroups of patients, as shown in Table 1:
- Group 1 -: Patients with normal levels of anti-dsDNA antibodies and normal concentrations of TBP-I (9 patients) or TBP-II (11 patients).
- Group 2 -: Patients with normal levels of anti-dsDNA antibodies but elevated concentrations of TBP-I (18 patients) or TBP-II (16 patients).
- Group 3 -: Patients with elevation of all three parameters (11 patients).

Although both groups 2 and 3 exhibited increased TBP levels, they differed significantly not only by the extent of increase in antibodies to dsDNA, but also in other parameters of disease activity (Table I). Compared to group 2, group 3 had higher mean disease index (1.7±0.6 vs 2.4±0.8,p<0.02), lower complement C4 levels (9.4±4 vs 30±13 mg/dl, p<0.001) and a higher mean prednisone intake (20.7≡17.7 vs 9±9 mg/day, p<0.05).

The enhanced formation of TBP-I and TBP-II, which correspond to the soluble TNF receptors type I and type II, respectively, may constitute an antagonistic mechanism of the organism to antagonize the TNF's damaging effects in the autoimmune diseases. The detection of a sub-group of SLE patients in this study, in which there is significant elevation of the TBPs, yet only marginal increase in disease activity, is consistent with the notion that the TBPs can attenuate progression of this disease and an indication that the TBPs can be used as therapeutical agent in SLE.

### Reference example 4: Bioactivity of TBPs in the sera of SLE patients - Inhibition of TNF cytotoxicity

In order to evaluate the bioactivity of the serum TBPs, serum samples were tested by a TNF cytotoxicity assay. The cytocidal activity of TNF was determined using murine A9 cells as targets. The cells were seeded in 96-well microplates at a density of 20,000 cells/well. After 24 hours, the supernatants were decanted. The cells were placed on ice and rhuTNF (5 units/ml, 6X10⁷ units/mg protein) was applied alone or together with serum samples with or without added antibodies to the TBPs (described in published European patent applications 398327 and 412486) or with samples of purified TBPs isolated from human urine. After additional incubation on ice for 90 minutes, the samples were decanted and the plates rinsed twice with cold medium at 4°C. This was followed by addition of Dulbecco's Modified Eagle's Minimal Essential Medium (DMEM) containing 10% fetal calf serum and 25 mg/ml cycloheximide. Cell viability was determined 12 hours later by the neutral red uptake assay.

Serum examples of SLE patients were tested by the above assay and were shown to protect A9 cells from the cytocidal effect of TNF. The extent of inhibition correlated with that observed upon application of the purified TBPs from urine in amounts identical to those present in the sera. Rabbit antisera to the TBPs, which by themselves had no effect on the A9 cytotoxicity assay, blocked the inhibitory efect of the human sera on this assay, thus confirming the assumption that the inhibition of TNF bioactivity observed, was solely due to the bioactivity of the TBPs present in the sera. This indicates that the TBPs may be effective in neutralizing the bioactivity of TBF in vivo, being capable of protecting patients from damages caused by TNF in autoimmune diseases.

**TABLE 1**

| Group | 1 | 2 | 3 |
|---|---|---|---|
| TEP | Normal range | High | High |
| Anti- dsDHA Ab | Normal range | Normal range | High |
| TBP-I | | | |
| No. of Patients | 9 | 18 | 11 |
| TBP-I (ng/ml) | 0.94±0.14 | 2.15±0.89 | 2.17±0.86 |
| anti ds DNA Ab% | 10.2±6.62 | 5.53±6.04 | 53±25 |
| Disease Index | 1.33±0.5 | 1.64±0.6 | 2.42±0.82 |
| Prednison intake (mg/day) | 0 | 9±9 | 20.7±17.9 |
| Complement C3 | - | 126±34 | 67±36 |
| Complement C4 | - | 30±13.2 | 9±4.6 |

| TBP-II | | | |
|---|---|---|---|
| No. of Patients | 11 | 16 | 11 |
| TBP-II.ng/ml | 3.54±0.75 | 8.06±1.98 | 8.57±2.61 |
| anti ds DNA Ab% | 10.2±7 | 5.3±5.9 | 51±25 |
| Disease Index | 1.18±0.4 | 1.78±0.57 | 2.41±0.79 |
| Prednison intake (mg/day) | 0 | 7.6± 9.5 | 20.7±18.8 |
| Complement C3 | - | 124.8±32 | 67±36 |
| Complement C4 | - | 32.5±12.6 | 9±4 |

## Claims

1. Use of a Tumor Necrosis Factor Binding Protein (TBP), a salt, a functional derivative, a precursor or an active fraction thereof, or combinations of the foregoing, for the manufacture of a pharmaceutical composition for the treatment of multiple sclerosis.

2. Use according to claim 1, wherein the TBP is TBP-I, TBP-II, or combinations thereof.

3. Use according to claim 1 or 2, wherein the TBP is natural TBP-I.

4. Use according to claim 1 or 2, wherein the TBP is recombinant TBP-I.

5. Use according to claim 1 or 2, wherein the TBP is natural TBP-II.

6. Use according to claim 1 or 2, wherein the TBP is recombinant TBP-II.

## Patentansprüche

1. Verwendung eines Tumornekrosefaktor-Bindungsproteins (TBP), eines Salzes, eines funktionellen Derivats, eines Vorläufers oder eines aktiven Teils davon, oder Kombinationen der vorgenannten, für die Herstellung eines Arzneimittels zur Behandlung von Multipler Sklerose.

2. Verwendung nach Anspruch 1, wobei das TBP TBP-I, TBP-II oder Kombinationen davon ist.

3. Verwendung nach Anspruch 1 oder 2, wobei das TBP natürliches TBP-I ist.

4. Verwendung nach Anspruch 1 oder 2, wobei das TBP rekombinantes TBP-I ist.

5. Verwendung nach Anspruch 1 oder 2, wobei das TBP natürliches TBP-II ist.

6. Verwendung nach Anspruch 1 oder 2, wobei das TBP rekombinantes TBP-II ist.

## Revendications

1. Utilisation d'une protéine de liaison du facteur de nécrose de tumeur (TBP), d'un sel, d'un dérivé fonctionnel, d'un précurseur ou d'une fraction active de cette protéine, ou de combinaisons des éléments précédents, pour la fabrication d'une préparation pharmaceutique pour le traitement de la sclérose en plaques.

2. Utilisation selon la revendication 1, dans laquelle la TBP est TBP-I, TBP-II ou leurs combinaisons.

3. Utilisation selon la revendication 1 ou 2, dans laquelle la TBP est la TBP-I naturelle.

4. Utilisation selon la revendication 1 ou 2, dans laquelle la TBP est la TBP-I recombinante.

5. Utilisation, selon la revendication 1 ou 2, dans laquelle la TBP est la TBP-II naturelle.

6. Utilisation selon la revendication 1 ou 2, dans laquelle la TBP est la TBP-II recombinante.
